# EUROPEAN PATENT SPECIFICATION

(11) **EP 0 617 632 B1**
(45) Date of publication and mention of the grant of the patent: **10.06.1998**
(21) Application number: 93921506.7
(22) Date of filing: 13.09.1993
(51) Int. Cl.: A61M 29/00

(54) **DETACHABLE EMBOLIC COIL ASSEMBLY**
ANORDNUNG EINER LÖSBAREN EMBOLIESPIRALFEDER
ENSEMBLE FIL HELICOIDAL EMBOLIQUE LIBERABLE

(30) Priority: 22.09.1992 US 949094; 19.04.1993 US 49577
(43) Date of publication of application: 05.10.1994
(60) Divisional application: 97120309.6
(73) Proprietor: TARGET THERAPEUTICS, INC., Fremont, CA 94537-5120 (US)
(72) Inventor: PALERMO, Thomas, J., San Jose, CA 95112 (US); GIA, Son, San Jose, CA 95111 (US)
(74) Representative: Price, Nigel John King
(86) International application number: US9308581
(87) International publication number: WO9406503

(56) References cited:
- WO-A-92/14408
- WO-A-93/11825
- US-A- 4 739 768
- US-A- 4 813 934
- US-A- 4 884 579
- US-A- 4 994 069
- US-A- 5 108 407
- US-A- 5 109 867
- US-A- 5 122 136

## Description

This invention is a surgical instrument and specifically is a device for delivering embolic coils to a selected site within the vasculature or other lumen of a human body via use of a catheter. The invention further includes the coils. In particular, the device (typically a "pusher assembly" in conjunction with a catheter) uses embolic coils having interlocking ends, e.g., clasps or hooks, on at least one end of the coils. The coils may further be secured to each other by a control wire within the catheter. The coils are pushed out the end of the catheter for placement and retraction of the optional control wire into the catheter body uncouples the distal coil. If no control wire is used, the coil is self-disengaging.

The endovascular treatment of a variety of vascular maladies throughout the body is an increasingly more important form of therapy. Catheters have been used to place various treatment materials, devices, and drugs within arteries and veins in the human body. Examples of these devices and their use in such treatments are shown in US-A-5,234,437 ("Detachable Pusher-Vasoocclusive Coil Assembly with Threaded Coupling") and US-A-5,261,916 published after the filing date of the present application ("Detachable Pusher-Vasoocclusive Coil Assembly with Interlocking Ball and Keyway Coupling"). These show methods and devices for delivery of coils or wires within the human body to sites such as aneurysms, to occlude those sites. Coils such as are discussed in US-A-4,994,069, may be of a regular or helical configuration or assume a random convoluted configuration at the selected site. The coils normally are made of a radiopaque, biocompatible metal such as platinum, gold, tungsten, or alloys of these and other metals.

In treating aneurysms it is common to place a number of coils within the aneurysm. The coils occlude the site by posing a physical barrier to blood flow and by promoting thrombus formation at the site.

Coils have typically been placed at the desired site within the vasculature using a catheter and a pusher. The site is first accessed by the distal end of a catheter. In treating peripheral or neural conditions requiring occlusion, the sites are accessed with flexible, small diameter catheters such as those shown in US-A-4,739,768 and US-A-4,813,934. The catheter may be guided to the site through the use of guidewires (see US-A-4,884,579) or by flow-directed means such as balloons placed at the distal end of the catheter. Use of guidewires involves the placement of relatively long, torqueable proximal wire sections within the catheter, which proximal sections are attached to more flexible distal end wire section designed to be advanced across sharp bends at vessel junctions. The guidewire is visible using x-ray and allows a catheter to be manipulated through extremely tortuous vessels, even when such vessels are surrounded by soft tissue such as the brain.

Once the selected site has been reached, the catheter lumen is cleared by removing the guidewire (if a guidewire has been used), and the coil is placed into the proximal open end of the catheter and advanced through the catheter with a pusher. Pushers are wires having a distal end that is adapted to engage and push the coil through the catheter lumen as the pusher is advanced through the catheter. When the coil reaches the distal end of the catheter, it is discharged from the catheter by the pusher into the vascular site. This technique of discharging the coil from the distal end of the catheter has a number of undesirable limitations. First, because of the plunging action of the pusher and the coil, the positioning of the coil at the site cannot be controlled to a fine degree of accuracy. Second, once the coil has left the catheter, it is difficult to reposition or retrieve the coil if such is desired. Nevertheless, the technique has the benefit of delivering multiple coils at low cost with a short delivery time.

Several techniques have been developed to enable more accurate placement of coils within a vessel. In one technique (US-A-5,122,136, on which is based the preamble of claim 1) the coil is bonded via a metal-to-metal joint to the distal end of the pusher. The pusher and coil are made of dissimilar metals. The coil-carrying pusher is advanced through the catheter to the site and a low electrical current is passed through the pusher-coil assembly. The current causes the connection between the pusher and the coil to be severed via electrolysis. The pusher may then be retracted leaving the detached coil at an exact position within the vessel. In addition to enabling more accurate coil placement, the electric current may facilitate thrombus formation at the coil site. The only perceived disadvantage of this method is that the electrolytic release of the coil requires a period of time so that rapid detachment of the coil from the pusher does not occur.

Another technique for detaching an embolic coil is shown in US-A-5,261,916. In that document, a coil having an enlarged portion is mated with a pusher having a keyway adapted to receive the enlarged portion of the coil in an interlocking relationship is covered by a coaxial member about the pusher and the coil. The coaxial member is movable by sliding the member axially. As the coaxial member is moved away from the junction where the coil's member engages the member of the keyway of the pusher, the coil disengages and the pusher is removed.

Another device for placement of coils is shown in US-A-5,234,437. This device includes a coil having a helical portion at one end and a pusher which is threaded to the inside of the helical coil by the use of a threaded section on the outside of the pusher. The device operates to release the coil by engaging the proximal end of the coil with a sleeve while the pusher is unthreaded. Once the pusher is free, the sleeve may be used to push the coil out into the treatment area.

Another method of placing an embolic coil is shown in US-A-5,108,407. This patent shows the use of a device in which embolic coils are separated from the distal end of a catheter by the use of heat-releasable adhesive bonds. The coil adheres to the therapeutic device via a mounting connection using a heat sensitive adhesive. Laser energy is transferred through a fiber optic cable, which cable terminates at the connector. The connector becomes warm and releases the adhesive bond between the connector and the coil.

None of these disclosed devices suggest coils having interlocking ends which allow an embolic coil to be positioned within a vessel and then released upon ejection of the coil from the catheter distal end or, optionally, upon retraction of a control wire positioned within that interlocking end.

According to a first aspect of the present invention there is provided a detachable coil assembly for use in occluding a selected vascular site, comprising a coil with a coil axis, a distal end, a proximal end and a cap on at least one of said proximal and distal ends, characterised in that the cap has an open receiving slot generally perpendicular to the coal axis, where the open receiving slot is suitable for accepting a hook adapted to enter the open receiving slot and adapted also to exit the open receiving slot.

According to a second aspect of the present invention there is provided a combination pusher assembly-coil assembly for use in occluding a selected vascular site, comprising:
(a) a coil assembly comprising a coil with an axis, a distal end, and a proximal end and having, on at least its proximal end, an open receiving slot generally perpendicular to the coil axis, where the open receiving slot is suitable for accepting a closed hook adapted to enter the receiving slot and adapted also to exit the open receiving slot; and
(b) a pusher assembly comprising a core wire having proximal and distal ends and adapted to fit within a catheter sheath and having a closed hook located at its distal end, said hook being adapted to enter and exit the open receiving slot in the proximal end of said coil.

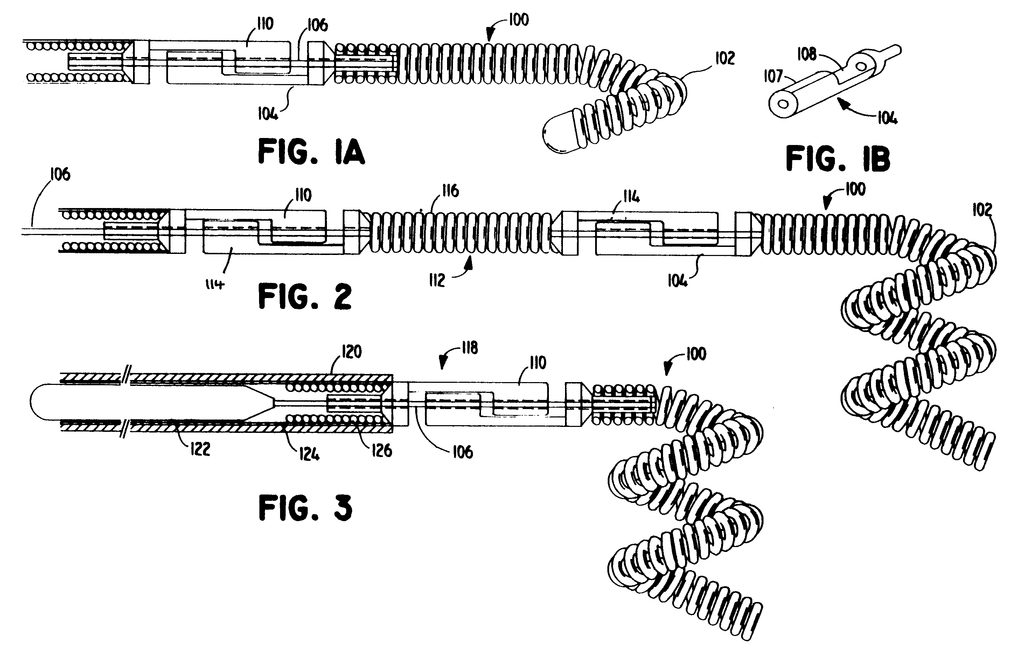

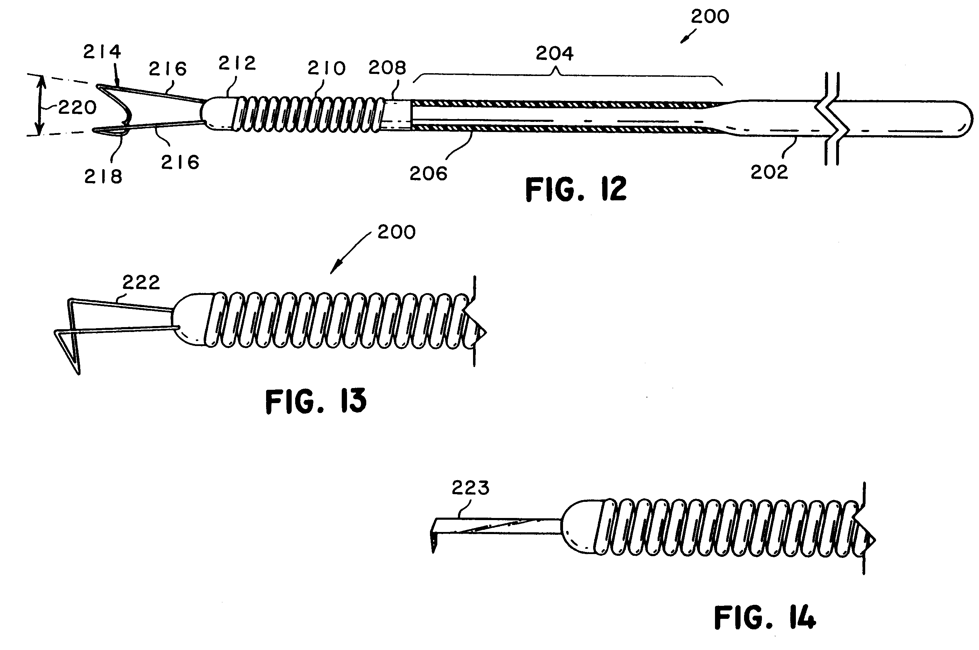

Embodiments of the invention will now be described, by way of example only, with reference to the accompanying drawings, described below. In the drawings, Figures 1-11 illustrate embodiments of apparatus not in accordance with the present invention. These drawings and their associated description are retained to assist in understanding the construction and functioning of the embodiments of apparatus which are in accordance with the present invention and which are illustrated in Figures 12-18.

Figures 1A and 1B show, respectively, a partial sectional view of a pusher assembly and an engaged coil assembly having an interlocking clasp at only one end and a front three-quarters view of one variation of the interlocking clamp.

Figure 2 shows a series of coil assemblies having either one or two interlocking clasps at their ends.

Figure 3 shows deployment of the interlocking coil within a catheter.

Figures 4 and 5 show the operation of the assembly as it places a coil within a target site.

Figures 6A and 6B show, respectively, a partial sectional view of a pusher assembly and an engaged coil assembly having a variation of an interlocking clasp and a front three-quarter view of that variation of the interlocking clasp.

Figures 7A, 7B, and 7C show a method of attaching coils having the interlocking clasp shown in Figures 6A and 6B to a pusher body within the catheter lumen.

Figure 8 shows a variation in which both the coils and the pusher body have simple loops as interlocking clasps.

Figure 9A shows a side view of a clasp, joinable to a coil or pusher, similar in shape to the Figure 1A to 5 clasp but, in design without, without the control wire.

Figure 9B shows a front three-quarter view of the Figure 9A clasp.

Figure 10A shows a side view of a clasp similar to the clasp shown in Figures 9A and 9B but with a ramped end.

Figure 10B shows a front three-quarter view of the Figure 10A clasp.

Figure 11 shows the Figure 9A clasp mounted on a pusher and on a coil as would be seen in a catheter.

Figure 12 shows in partial cross-sectional view, a guidewire pusher assembly having a W-shaped hook at the distal end for engaging coils having a cooperatively shaped slot.

Figure 13 shows the distal tip of a variation of the guidewire pusher shown in Figure 12 but having a simply V-shaped hook positioned at the distal end.

Figure 14 shows the distal tip of a variation of the guidewire pusher shown in Figure 12 but having a solid hook positioned at the distal end.

Figure 15 shows an embolic coil having the desired engaging slot at one end.

Figure 16 shows a coil similar in construction to that found in Figure 15, but also having a hook at its other end.

Figure 17 shows an embolic coil similar to that in Figure 15 and a pusher similar to Figure 14 using a control wire to allow disengagement of the coil.

Figure 18 depicts the manner in which the invention operates.

The coil assembly (100) is shown in Figure 1. The coil (102) is shown as helical in form, although it may be any other suitable form. The coil should be of a size sufficiently small that it may be advanced through a catheter that is appropriately sized for accessing the targeted vascular site. For instance, when accessing a brain aneurysm in a small vessel, an appropriately sized catheter is quite small and very flexible. The coil in such a situation must be small enough to fit through the catheter and out its distal end at the treatment site.

The coil is desirably made up of a radiopaque, physiologically compatible material. For instance, the material may be platinum, gold, tungsten, or alloys of these. Certain polymers are also suitable as coil material either alone or in conjunction with metallic markers providing radiopacity. These materials are chosen so that the procedure of locating coils within the vessel may be viewed using radiography. However, it is also contemplated that these coils may be made of various other biologically inert polymers or of carbon fiber.

The size of the coil and its constituent winding will depend upon the use to which the coil will be placed. For occluding peripheral or neural sites, the coils will typically be made of 0.05 to 0.15 mm diameter wire (platinum or platinum/tungsten alloy) that may be wound to have an inner diameter of 0.15 to 1.5 mm with a minimum pitch -- that is to say that the pitch is equal to the diameter of the wire used in the coil. The outer diameter is then typically between 0.25 mm to 1.8 mm. The length of the coil will normally be in the range of 0.5 to 60 cm, preferably 0.5 to 40 cm.

If desired, the coil may be formed in such a way that the coil is essentially linear as it passes through the catheter and yet assumes a randomly oriented relaxed condition after it is released from the distal end of the catheter. A discussion of this variation may be found in US-A-4,994,069.

Fixedly attached to coil (102), as is shown in Figure 1A, is interlocking clasp (104). Interlocking clasp (104) as is depicted in the front three-quarter view in Figure 1B, has an interior passageway allowing the control wire (106) to pass completely therethrough. As is shown in Figure 1A, the male portion of the next adjacent interlocking clasp (110) fits into the area (108) left within clasp (104) so to allow the interlocking to take place. Said another way, the distal portion of interlocking clasp (104) is generally cylindrical in shape but has a surface (107), which may be cut or milled away, allowing the portion to mesh within the middle area (108) of an adjacent clasp. The proximal section is adapted for attaching to a coil or to a pusher assembly. The attachment may be by welding, soldering, gluing, or the like. With a control wire (106) passing through the axis of both interlocking clasps (104) and (110), the two are locked together. As is shown in Figure 1A, the control wire may extend through the length of coil (102).

Figure 2 shows an intermediate coil assembly (100) comprising coil (102) and interlocking clasp (104) (joined with coil assembly (112)) which has interlocking clasp (114) fixedly attached at both ends of the intervening coil (116). As was the situation in Figure 1A, the proximal interlocking clasp (114) is joined by control wire (106) with interlocking clasp (110). In this way, a significant number of coils (112) may be loaded onto a control wire (106) and delivered to the treatment site without removal of the control wire from the catheter.

Figure 3 shows the relationship of coil assembly (100) and the pusher assembly (118) with its distal interlocking clasp (110) as it fits within catheter sheath (120). Also shown is movable inner core member (122) and the sheath (124) which fits within catheter sheath (120) and supports interlocking clasp (110). Shown in Figure 3 is the stiffener spring (126) which provides form and support for the distal end of the pusher assembly (118) and in particular rigidly adheres to interlocking clasp (110). Inner core member (122) allows the control wire (106) to be moved axially along the interior of the catheter sheath (120) and the pusher assembly (118). Movement of the inner core member (122) in a proximal direction permits uncoupling of the coil as will be discussed in more detail below.

The length of pusher assembly (118) will be such as to be capable of being advanced entirely through the catheter to place coil (102) at the target site but yet with a sufficient portion of the proximal end of the pusher assembly (118) protruding from the proximal end of the catheter to enable the control wire (106) to be manipulated. For use in peripheral or neural surgeries, the pusher will normally be about 100-200 cm in length, more normally 130-180 cm in length. The diameter of the pusher assembly (118) is usually in the range of 0.25 to about 0.90 mm.

As indicated previously, conventional catheter insertion and navigational techniques involving guidewires or flow-directed devices may be used to access the site with a catheter. Once the distal end of the catheter is positioned at the site, often by locating its distal end through the use of radiopaque marker material and radiography, the catheter is cleared. For instance, if a guidewire has been used to position the catheter, it is withdrawn from the catheter and then the pusher assembly (118) having coil assembly (100) at the distal end is advanced through the catheter. The pusher assembly (118) is advanced past the distal end of the catheter so that the coil is free of the catheter and with the coil positioned precisely at the desired treatment site.

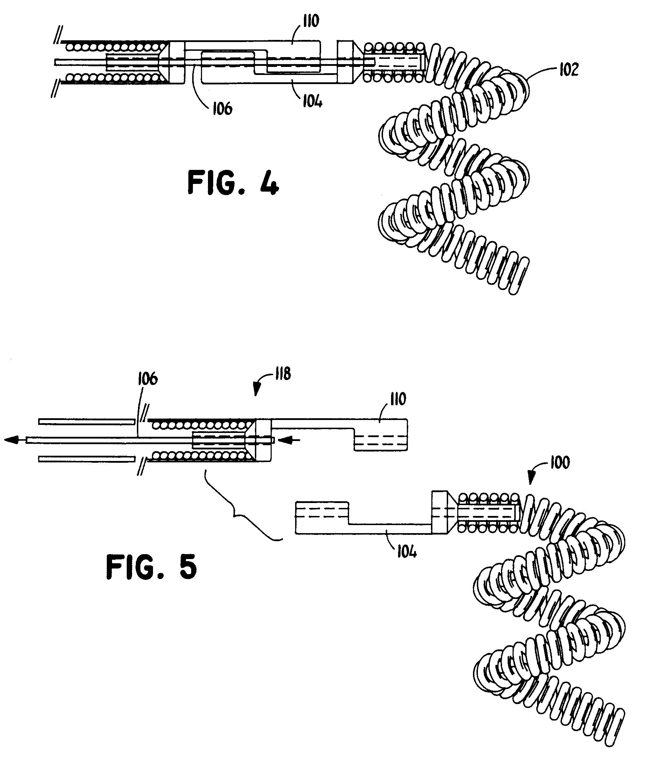

As is shown in Figures 4 and 5, control wire (106) is withdrawn from the junction between coil interlocking clasp (104) and the other interlocking clasp (110). Coil assembly (100) is then free. The entire catheter may then be removed or the pusher assembly (118) may be withdrawn from the catheter lumen to provide for installation of other coils. If additional coils are to be placed at the target site, the procedure is repeated. After the desired number of coils have been placed at the site, the catheter is withdrawn from the vessel.

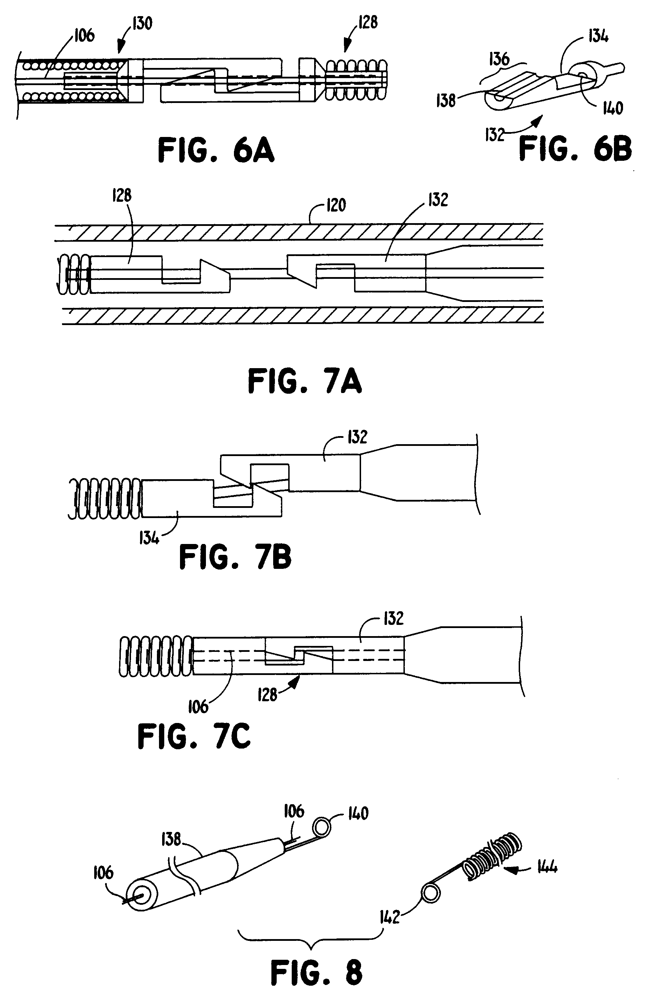

Figure 6A shows a variation in which coil assembly (128) is interlocked with pusher assembly (130) by control wire (106). The depicted coil assembly (128) and pusher assembly (130) are different in that they incorporate the interlocking clasp (132) design shown more clearly in Figure 6B. The interlocking clasp (132), as with the clasp depicted in Figure 1B, utilizes an open area (134) within the clasp (132) to accept the mating ramp latch (136) from another similar clasp. The ramp latch (136) typically has a slot (138) and a passageway (140) to permit passage of the control wire through the clasp (132) from end to end without obstruction.

The ramp latch (136) allows easy assembly of a string of coils within the catheter for subsequent placement using the device.

Such an assembly process is shown in Figures 7A, 7B, and 7C.

Figure 7A shows a pusher assembly (130) approaching a coil assembly (128) which has been previously placed within a catheter sheath (120). The distal interlocking clasp (132) on the pusher assembly (130) is positioned to interlock with the proximal interlocking clasp (134) on the coil assembly (128).

Figure 7B shows the two interlockng clasps (132 and 134) as they approach their respective ramps contacting and causing the two clasps to displace axially within the catheter sheath.

Figure 7C shows the location of the coil assembly (128) and the pusher assembly (132) after the respective clasps are interlocked and the control wire (106) has been placed through the passageways within the clasps.

Figure 8 shows an elegantly simple variation in which the pusher (138) is a tubing member having a control wire (106) within its core. The clasp portion (140) is a simple loop comprising, e.g., wire or small rod. The corresponding interlocking loop (142) on the coil (144) forms the junction with the clasp on the pusher.

The variation of shown in Figures 6A, 6B, 7A, 7B, 7C, and 8 may be placed within the vasculature in the same manner as shown for the variation shown in Figs. 4 and 5.

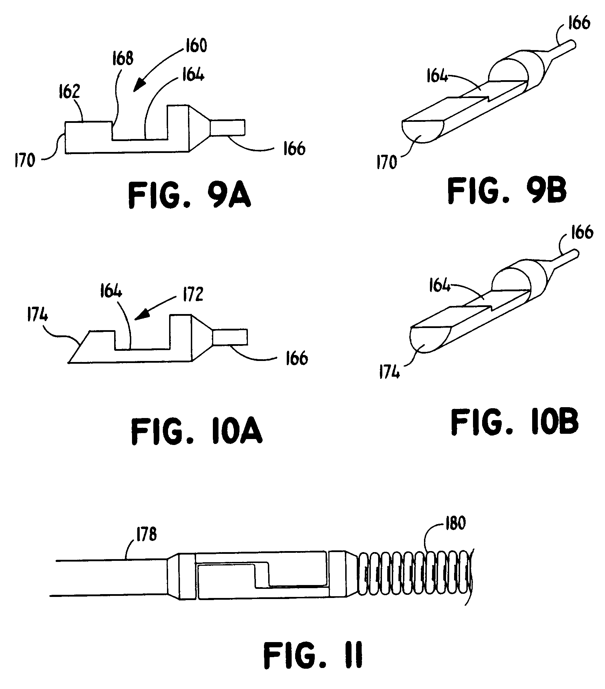

Figure 9A shows a side view of a clasp (160) similar in design to the clasp discussed in conjunction with Figures 1A, 1B, 2, 3, 4, and 5. In contrast to the clasp found on those vasooclusive devices, the Figure 9A clasp (160) contains no lumen therethrough for a control wire. As was the case with the clasp above, the clasp is generally cylindrical in shape but has a surface (162) which may be cut or milled away to allow it to mesh with a receiver area (164) in the next adjacent clasp as is shown in Figure 11. The other end of the clasp is adapted to allow joining with the end of a coil or pusher. Although that other end is shown with a reduced diameter (166), to allow insertion of the end (166) into the coil or pusher, other end shapes are certainly appropriate, e.g., helical to accept the coil, square, bulbed, etc., the reduced diameter cylinder is very desirable. The vertical or mating surface (168) pulls the next device in the chain along when it contacts the similar surface in that next device. The end surface (170) pushes against the next device when in a chain.

Figure 9B shows a front, elevated, three-quarter view of the clasp found in Figure 9A. Figure 9B shows the end surface (170), the receiver area (164), and the reduced diameter shaft (166) for mating with the coil or pusher.

Figure 10A shows a clasp (172) much like that shown in Figure 9A except that the end surface is a ramp (174) to permit assembly ease in placing the coils in the catheter or introducer.

Figure 10B shows a front, elevated, three-quarter view of the clasp found in Figure 10A. Figure 10B shows the end ramp (174), the receiver area (164), and the reduced diameter shaft (166) for mating with the coil or pusher.

Figure 11 shows how the clasps found in Figures 9A and 9B mesh when installed on a pusher (178) or a coil (180). It is within the scope of this variation that the clasp be mounted on the ends of pushers, coils (one or both ends), and that multiple coils or other vasooclusive devices be joined in multiple end-to-end trains for introduction into the vasculature.

An embodiment of pusher assembly (200) that is in accordance with the present invention is shown in Figure 12. The configuration of the body of the pusher assembly (200) is not particularly critical, and many variations known in the art would likely be suitable. The variation shown here entails, at the distal end, a stainless steel core (202) having a small diameter section (204) covered by a desired polymeric material (206) such as tetrafluoroethylene, or other suitable fluorinated hydrocarbon polymers; hydrophilic polymers such as polyvinylpyrrolidone, polyethyleneoxide, or polyhydroxyethylmethacrylate, or copolymers, or mixtures, or blends thereof; or various silicone-based polymeric materials; or polyolefins such as polyethylene, polypropylene, or their copolymers, mixtures, or blends; or appropriate polyurethane polymers. This coating provides a slippery surface allowing ease of insertion and traverse through the catheter body.

It is desirable to include a radiopaque marker (208). Such markers are common in this art and may be made of known radiopaque materials such as platinum, palladium, or other such materials. Commonly, the radiopaque marker (208) is a coil which is brazed or soldered to the guidewire and may be coated with the polymeric materials (206). This marker allows the tending physician to monitor the progress of the guidewire tip via fluoroscopy and, obviously, allow proper placement of the coil which is attached to the end of the pusher guidewire (200).

More distal of the radiopaque marker (208) may be found a flexible coil (210). This coil covers a tapered section of the core wire (202). Tapering the inner wire and enclosing it in a wire coil increases the column strength of the tapered wire section without significant loss of flexibility and increases the radial capacity of the guidewire to allow fine manipulation of the guidewire through various tortuous portions of the vasculature. The tip of the core wire (202) and the distal portion of the wire coil (210) are typically joined by use of a solder joint (212). To this point, the guidewire is of a typical guidewire respected in this art. See, for instance, those guidewires shown in US-A-3,789,841; US-A-4,545,390; and US-A-4,619,274.

Unique to this variation of the invention is the hook (214) placed at the most distal end of the guidewire assembly (200) which transforms it into a pusher.

Engaging hook (214) has two legs (216) which are based in solder joint (212). The outer hook portion (218) is configured so that it slides into the conforming slot in the coils as discussed below. The diameter (220) of the hook (214) is typically no larger than the inside diameter of the catheter assembly into which it is placed. Obviously, if the diameter is larger, it will bind in the catheter and be of little use. The most distal portion of the hook (218) is configured in such a way that the "W" portion is in a plane which is generally perpendicular to the longitudinal axis of the guidewire pusher assembly (200). The engaging hook (214) need be made only of a material which is adequate under the circumstances of use. For instance, the hook may be of a stainless steel wire which may be soldered onto the end of the guidewire assembly (200) and bent into desirable shape. In this way, the hook may be used to push the attached coil through the catheter without bending. The length of guidewire pusher assembly (200) should be such as to be capable of being advanced entirely through a catheter to place a coil such as discussed below at the target site, but yet retain a sufficient portion of the proximal end of the guidewire pusher assembly (200) protruding from the proximal end of the catheter to enable the pusher to be manipulated. For use in peripheral and neural surgeries, the pusher will normally be about 100-200 cm in length, more normally 130-180 cm in length. The diameter of the guidewire pusher assembly (100) is usually in the range of 0.25 to about 0.90 mm.

Figure 13 shows a variation of the distal tip of guidewire pusher assembly (200) having a slightly different configuration than that shown in Figure 12. In this instance, the hook is a simple "V"- or "U"-shaped hook which also will engage with the slotted coils described below. The materials of construction and other such variables are similar to those for the hook of Figure 12. Figure 14 shows an additional variation of the pusher assembly (200) having a hooked distal tip. This hook (223) is of a bent ribbon rather than the wire configuration shown in Figures 12 and 13. The materials of construction and method of attachment are similar to those used in the Figure 12 and 13 variations. The ribbon is bent in such a way as to allow insertion of the hook's bent lip into the slot found in the coils discussed below.

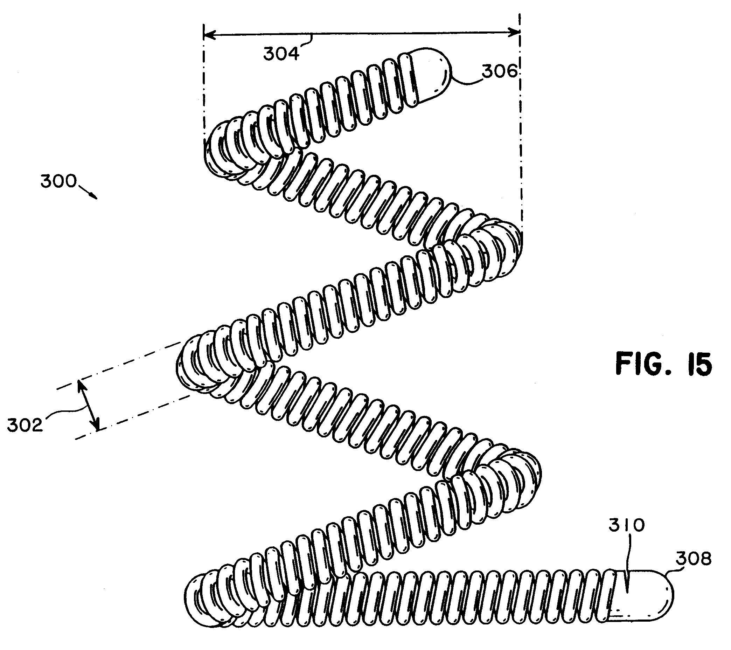

The coil typical of that which might be used with this invention, is shown in Figure 15. The coil (300) is shown as helical in form, although it may be any other suitable form. The coil shown is one having a primary and a secondary diameter. The primary diameter (302) is sufficiently small that the coil (300), when straightened, would fit inside the lumen of the catheter assembly. The coil assembly shown assumes a second diameter (304) when ejected from the tip of the catheter using the pusher guidewire (200) shown in Figure 12.

Coil (300) may be made up of the same or similar radiopaque, physiologically compatible materials discussed in relation to coil (100) applicable to Figures 1-5 above. The size, length, diameter (inner and outer), pitch, and configuration all may be as discussed above.

Whatever the configuration may be, the coil typically has caps at each end. Specifically, the distal end of the coil (300) will have a distal cap to (306) which may be solder or epoxy or other filling adhesive or fused from the coil metal, preferably forming a rounded form to prevent the coil from hanging up within the catheter or an inappropriate place within the patient's vasculature. The unique aspect of this invention is found at the proximal end of the coil (308). The proximal end typically will be soldered or glued, much in the way that the distal end has been, but is configured in such a way that a slot (310) is opened during the soldering or gluing process and will accept the hook, variously (214) in Figure 12 or (222) in Figure 13 or (223) in Figure 14 into the slot. Obviously, the receiving slot (310) may be generally substantially perpendicular to the local axis of the coil.

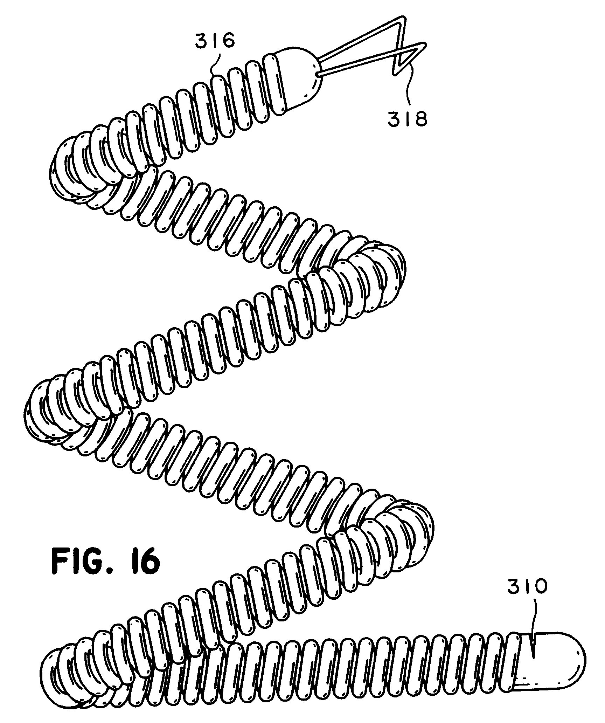

Figure 16 depicts a variation of the coil shown in Figure 15. This variation, however, includes, at the distal end of the coil (316), a hook (318) of configuration similar to that found in discussing the guidewire pusher assemblies in Figures 12 and 13. This configuration allows the introduction of discrete segments of coils into the catheter and separate placement of them should such a situation be desirable. In such an instance, the hook (318) would be introduced into the receiver slot (310) in the similar coil next in line. The most proximal of the coils would, in turn, be engaged with a hook on a pusher assembly such as (200) shown in Figure 12.

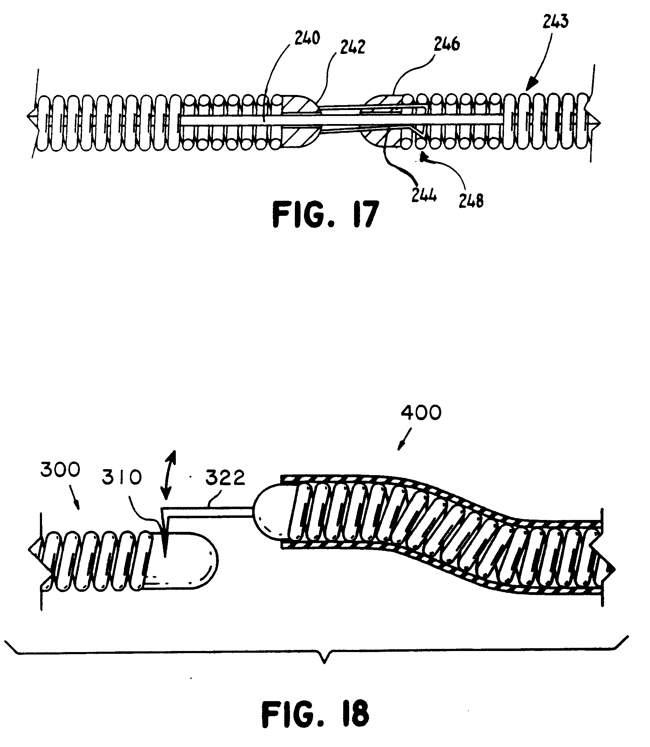

Figure 17 shows a variation of the invention shown in Figures 12-16 in which a control wire (240) is placed generally through an axial passageway between the distal (or proximal) end (242) of a coil or pusher having a hook (244) and the corresponding end (246) of a coil (243) having a slot (248) cooperating with the hook (244). As with the variation shown in Figures 1-8, the control wire (240) may be withdrawn when the coil (243) is situated at the selected vascular site to disengage the coil (243). Use of the control wire permits more precise placement of coil (243) and its gentle disengagement.

Figure 18 is a side view depicting how the hook (322), as depicted here, is placed in slot (310) of the coil assembly (300). The tip of a typical catheter (400) is shown in the Figure. Again, the overall diameter of the various assemblies as put together for introduction into or out of catheter must be of a diameter smaller than the diameter lumen in catheter (400). Obviously, too large a coil/pusher combination will not be particularly valuable in a situation where such is needed.

As indicated previously, conventional catheter insertion and navigational techniques involving guidewires or even flow-directed devices may be used to access a chosen vascular site with a catheter. Once the distal end of the catheter is positioned at that chosen site, often by locating its distal end through the use of a radiopaque marker material and radiography, the catheter is cleared. For instance, if a guidewire has been used to position a catheter, it is withdrawn from the catheter and then the guidewire pusher assembly such as (200) shown in Figure 12 having coil assembly such as (300) in Figure 15 is assembled and introduced into the proximal end of the catheter. The guidewire pusher assembly is then advanced so that its distal end is free of the distal end of the catheter and the coil positioned precisely at the desired site. The pusher assembly (200) may require a twisting movement to free the distal hook from the receiving slot in the coil.

## Claims

1. A detachable coil assembly for use in occluding a selected vascular site, comprising a coil (300,316) with a coil axis, a distal end, a proximal end (308) and a cap on at least one of said proximal and distal ends, characterised in that the cap has an open receiving slot (310) generally perpendicular to the coil axis, where the open receiving slot (310) is suitable for accepting a hook (214,222,223, 318,322) adapted to enter the open receiving slot and adapted also to exit the open receiving slot.

2. The assembly of claim 1, where the distal coil end comprises a closed hook (318) adapted to enter a receiving slot (310) in another detachable coil assembly.

3. The assembly of claim 1, where the distal coil end comprises a bent ribbon having a lip adapted to enter a receiving slot (310) in another detachable coil assembly.

4. The assembly of any one of claims 1 to 3, additionally comprising an axial passageway extending from the distal to the proximal end.

5. A combination pusher assembly-coil assembly for use in occluding a selected vascular site, comprising:
(a) a coil assembly comprising a coil (243,300,316) with an axis, a distal end, and a proximal end and having, on at least its proximal end, an open receiving slot (248,310) generally perpendicular to the coil axis, where the open receiving slot (248,310) is suitable for accepting a closed hook (214,222,223,244) adapted to enter the receiving slot and adapted also to exit the open receiving slot; and
(b) a pusher assembly (200) comprising a core wire (202) having proximal and distal ends and adapted to fit within a catheter sheath and having a closed hook (214, 222,223,244) located at its distal end, said hook being adapted to enter and exit the open receiving slot (248,310) in the proximal end of said coil (243,300,316).

6. The assembly of claim 5, additionally comprising a catheter sheath disposed about the pusher assembly (200) and coil (300,316).

7. The assembly of claim 5 or claim 6, where the hook is a closed wire hook (214,222,223,244) adapted to enter said open receiving slot (248,310) in the proximal end of the coil (243,300,316).

8. The assembly of claim 5 or 6, where the hook is a bent ribbon (223) having a lip adapted to enter said open receiving slot (310) in the proximal end of the coil (300,316).

9. The assembly of any one of claims 5 to 7, additionally comprising a control wire (240) for extending through an axial passageway between the distal end (242) of the pusher having the hook (244) and the proximal end (246) of the coil (243) having the open receiving slot (248) to help secure the coil (246) to the pusher when the hook (244) is engaged in the open receiving slot (248), and wherein withdrawal of the control wire (240) from the coil (243) will permit disengagement of the coil (243) from the pusher.

10. The assembly of any one of the preceding claims, where the coil axis length is 0.5 to 100 cm.

11. The assembly of any one of the preceding claims, where the coil outer diameter is between 0.25 mm and 1.8 mm.

12. The assembly of any one of the preceding claims, where the coil (300,316) is a helical coil.

13. The assembly of any one of the preceding claims, comprising a plurality of coils.

14. The assembly of any one of the preceding claims, where the coil (300,316) has a random or straight configuration.

## Patentansprüche

1. Abtrennbare Spulenanordnung zur Verwendung beim Verschließen einer ausgewählten Gefäßstelle, die eine Spule (300, 316) mit einer Spulenachse, einem distalen Ende, einem proximalen Ende (308) und einer Kappe auf mindestens einem der proximalen und distalen Enden umfaßt,
dadurch gekennzeichnet, daß die Kappe einen offenen Aufnahmeschlitz (310) umfaßt, der sich im allgemeinen senkrecht zu der Spulenachse erstreckt, wobei der offene Aufnahmeschlitz (310) dafür geeignet ist, einen Haken (214, 222, 223, 318, 322) aufzunehmen, der so ausgelegt ist, daß er in den offenen Aufnahmeschlitz eindringen kann, und auch so ausgelegt ist, daß er den offenen Aufnahmeschlitz verlassen kann.

2. Anordnung nach Anspruch 1, bei der das distale Spulenende einen geschlossenen Haken (318) umfaßt, der so ausgelegt ist, daß er in einen Aufnahmeschlitz (310) in einer anderen abtrennbaren Spulenanordnung eindringen kann.

3. Anordnung nach Anspruch 1, bei der das distale Spulenende einen abgebogenen Streifen umfaßt, der einen Rand aufweist, der so ausgelegt ist, daß er in einen Aufnahmeschlitz (310) in einer anderen abtrennbaren Spulenanordnung eindringen kann.

4. Anordnung nach einem der Ansprüche 1 bis 3, die zusätzlich einen axialen Durchgang umfaßt, der sich von dem distalen zu dem proximalen Ende erstreckt.

5. Eine Kombination aus einer Schiebevorrichtungsanordnung und einer Spulenanordnung zur Verwendung beim Verschließen einer ausgewählten Gefäßstelle, umfassend:
(a) eine Spulenanordnung, die eine Spule (243, 300, 316) mit einer Achse, einem distalen Ende und einem proximalen Ende umfaßt und zumindest an ihrem proximalen Ende einen offenen Aufnahmeschlitz (248, 310) umfaßt, der sich im allgemeinen senkrecht zu der Spulenachse erstreckt, wobei der offene Aufnahmeschlitz (248, 310) dafür geeignet ist, einen geschlossenen Haken (214, 222, 223, 244) aufzunehmen, der so ausgelegt ist, daß er in den Aufnahmeschlitz eindringen kann, und auch so ausgelegt ist, daß er den offenen Aufnahmeschlitz verlassen kann, und
(b) eine Schiebevorrichtungsanordnung (200), die einen Kerndraht (202) umfaßt, der proximale und distale Enden aufweist und so ausgelegt ist, daß er in eine Katheterumhüllung paßt, und einen geschlossenen Haken (214, 222, 223, 244) aufweist, der sich an ihrem distalen Ende befindet, wobei der Haken so ausgelegt ist, daß er in den offenen Aufnahmeschlitz (248, 310) in dem proximalen Ende der Spule (243, 300, 316) eindringen und diesen verlassen kann.

6. Anordnung nach Anspruch 5, die zusätzlich eine Katheterumhüllung umfaßt, die sich um die Schiebevorrichtungsanordnung (200) und die Spule (300, 316) herum befindet.

7. Anordnung nach Anspruch 5 oder Anspruch 6, bei der der Haken ein geschlossener Drahthaken (214, 222, 223, 244) ist, der so ausgelegt ist, daß er in den offenen Aufnahmeschlitz (248, 310) in dem proximalen Ende der Spule (243, 300, 316) eindringen kann.

8. Anordnung nach Anspruch 5 oder 6, bei der der Haken ein abgebogener Streifen (223) ist, der einen Rand aufweist, der so ausgelegt ist, daß er in den offenen Aufnahmeschlitz (310) in dem proximalen Ende der Spule (300, 316) eindringen kann.

9. Anordnung nach einem der Ansprüche 5 bis 7, die zusätzlich einen Steuerdraht (240) umfaßt, der sich durch einen axialen Durchgang zwischen dem distalen Ende (242) der Schiebevorrichtung, das den Haken (244) aufweist, und dem proximalen Ende (246) der Spule (243), das den offenen Aufnahmeschlitz (248) aufweist, erstrecken kann, um das Befestigen der Spule (246) an der Schiebevorrichtung zu unterstützen, wenn der Haken (244) mit dem offenen Aufnahmeschlitz (248) in Eingriff kommt, und wobei das Herausziehen des Steuerdrahts (240) aus der Spule (243) das Loskoppeln der Spule (243) von der Schiebevorrichtung erlaubt.

10. Anordnung nach einem der vorhergehenden Ansprüche, bei der die Spulenachsenlänge 0,5 bis 100 cm beträgt.

11. Anordnung nach einem der vorhergehenden Ansprüche, bei der der äußere Spulendurchmesser zwischen 0,25 mm und 1,8 mm beträgt.

12. Anordnung nach einem der vorhergehenden Ansprüche, bei der die Spule (300, 316) eine schraubenförmige Spule ist.

13. Anordnung nach einem der vorhergehenden Ansprüche, die eine Vielzahl von Spulen umfaßt.

14. Anordnung nach einem der vorhergehenden Ansprüche, bei der die Spule (300, 316) eine beliebige oder geradläufige Konfiguration aufweist.

## Revendications

1. Ensemble de serpentin détachable destiné à être utilisé pour occlure un site vasculaire sélectionné, comprenant un serpentin (300, 316) avec un axe de serpentin, une extrémité distale, une extrémité proximale (308) et un capuchon sur au moins l'une desdites extrémités proximale et distale, caractérisé en ce que le capuchon comporte une fente de réception ouverte (310) globalement perpendiculaire à l'axe de serpentin, la fente de réception ouverte (310) étant appropriée pour accepter un crochet (214, 222, 223, 318, 322) adapté pour entrer dans la fente de réception ouverte, et adapté également pour quitter la fente de réception ouverte.

2. Ensemble selon la revendication 1, dans lequel l'extrémité de serpentin distale comprend un crochet fermé (318) adapté pour entrer dans une fente de réception (310) dans un autre ensemble de serpentin détachable.

3. Ensemble selon la revendication 1, dans lequel l'extrémité de serpentin distale comprend un ruban incurvé comportant une lèvre adaptée pour entrer dans une fente de réception (310) dans un autre ensemble de serpentin détachable.

4. Ensemble selon l'une quelconque des revendications 1 à 3, comprenant de plus un passage axial s'étendant de l'extrémité distale à l'extrémité proximale.

5. Combinaison d'ensemble de poussoir et d'ensemble de serpentin destinée à être utilisée pour occlure un site vasculaire sélectionné, comprenant :
(a) un ensemble de serpentin comprenant un serpentin (243, 300, 316) avec un axe, une extrémité distale et une extrémité proximale, et comportant, au moins sur son extrémité proximale, une fente de réception ouverte (248, 310) globalement perpendiculaire à l'axe de serpentin, la fente de réception ouverte (248, 310) étant appropriée pour accepter un crochet fermé (214, 222, 223, 244) adapté pour entrer dans la fente de réception, et adapté également pour quitter la fente de réception ouverte ; et
(b) un ensemble de poussoir (200) comprenant un fil de coeur (202) comportant des extrémités proximale et distale et adapté pour s'adapter à l'intérieur d'un manchon de cathéter et comportant un crochet fermé (214, 222, 223, 244) disposé à son extrémité distale, ledit crochet étant adapté pour entrer dans la fente de réception ouverte (248, 310) dans l'extrémité proximale dudit serpentin (243, 300, 316) et quitter celle-ci.

6. Ensemble selon la revendication 5, comprenant de plus un manchon de cathéter disposé autour de l'ensemble de poussoir (200) et un serpentin (300, 316).

7. Ensemble selon la revendication 5 ou la revendication 6, dans lequel le crochet est un crochet en fil fermé (214, 222, 223, 244) adapté pour entrer dans ladite fente de réception ouverte (248, 310) dans l'extrémité proximale du serpentin (243, 300, 316).

8. Ensemble selon la revendication 5 ou 6, dans lequel le crochet est un ruban incurvé (223) comportant une lèvre adaptée pour entrer dans ladite fente de réception ouverte (310) dans l'extrémité proximale du serpentin (300, 316).

9. Ensemble selon l'une quelconque des revendications 5 à 7, comprenant de plus un fil de commande (240) destiné à s'étendre à travers un passage axial entre l'extrémité distale (242) du poussoir comportant le crochet (244) et l'extrémité proximale (246) du serpentin (243) comportant la fente de réception ouverte (248) afin d'aider à fixer le serpentin (246) au poussoir lorsque le crochet (244) est en prise dans la fente de réception ouverte (248), et dans lequel le retrait du fil de commande (240) depuis le serpentin (243) permet le désengagement du serpentin (243) d'avec le poussoir.

10. Ensemble selon l'une quelconque des revendications précédentes, dans lequel la longueur d'axe de serpentin est comprise entre 0,5 et 100 cm.

11. Ensemble selon l'une quelconque des revendications précédentes, dans lequel le diamètre extérieur du serpentin est compris entre 0,25 mm et 1,8 mm.

12. Ensemble selon l'une quelconque des revendications précédentes, dans lequel le serpentin (300, 316) est un serpentin hélicoïdal.

13. Ensemble selon l'une quelconque des revendications précédentes, comprenant une pluralité de serpentins.

14. Ensemble selon l'une quelconque des revendications précédentes, dans lequel le serpentin (300, 316) a une configuration aléatoire ou droite.
